# EUROPEAN PATENT SPECIFICATION

(11) **EP 4 637 759 B1**
(45) Date of publication and mention of the grant of the patent: **04.11.2026**
(21) Application number: 23834122.6
(22) Date of filing: 19.12.2023
(51) Int. Cl.: A61K 31/409, A61P 25/00

(54) **UROPORPHYRIN I FOR USE IN THE TREATMENT OF PRION DISEASES**
UROPORPHYRIN I ZUR BEHANDLUNG VON PRIONERKRANKUNGEN
UROPORPHYRINE I POUR LE TRAITEMENT DE MALADIES À PRIONS

(30) Priority: 20.12.2022 EP 22383243
(43) Date of publication of application: 29.10.2025
(73) Proprietor: Asociación Centro de Investigación Cooperativa en Biociencias - CIC bioGUNE, 48160 Derio (ES); Atlas Molecular Pharma, S.L., 48160 Derio - Bizkaia (ES)
(72) Inventor: CASTILLA CASTRILLÓN, Joaquín, 48160 Derio, Vizcaya (ES); ERAÑA LASAGABASTER, Hasier, 48160 Derio, Bizkaia (ES); MORENO CHARCO, Jorge, 48160 Derio, Bizkaia (ES); MILLET AGUILAR-GALINDO, Oscar, 48160 Derio, Vizcaya (ES); GARCÍA MARTÍNEZ, Sandra, 48160 Derio, Bizkaia (ES); BERNARDO-SEISDEDOS, Ganeko, 48160 Derio, Bizkaia (ES)
(74) Representative: ABG Intellectual Property Law, S.L.
(86) International application number: PCT/EP2023/086566
(87) International publication number: WO 2024/133215

(56) References cited:
- WO-A2-00/09111
- US-A1- 2006 172 986
- C. R TREVITT: "A systematic review of prion therapeutics in experimental models", BRAIN, vol. 129, no. 9, 1 September 2006 (2006-09-01), pages 2241 - 2265, XP055036135, ISSN: 0006-8950, DOI: 10.1093/brain/awl150

## Description

### Field of the Invention

The present invention relates to methods, compounds and compositions that can be used for the treatment and/or prevention of prion diseases (transmissible spongiform encephalopathies).

### Background of the Invention

Transmissible spongiform encephalopathies (TSE) or prion diseases are a group of rapidly progressing and invariably fatal neurodegenerative disorders affecting humans among other mammals and for which no treatment is available. In human beings, the best-known prion diseases include Creutzfeldt-Jakob diseases (CJD), fatal familial insomnia (FFI) and Gerstmann-Sträussler-Scheinker syndrome (GSS), whereas in other mammals, scrapie from sheep and goat, bovine spongiform encephalopathy from cattle, and chronic wasting disease from deer are the most common.

Among the main hallmarks of these diseases are the spongiform changes in the brain, intense vacuolation due to extensive neuronal death, and the presence of amyloid plaques or deposits, large proteic aggregates that accumulate in the central nervous system. Being the latter a common characteristic of amyloidogenic or protein misfolding-related neurodegenerative diseases. In the case of TSE, these protein deposits are composed almost exclusively by an aberrantly folded (misfolded) isoform of the endogenous prion protein also known as cellular prion protein or PrP^{C}. This is a normally cell membrane-bound glycoprotein that is most abundantly expressed in the cells from the central nervous system, the physiological role of which is not exactly understood, but is related according to the latest research with myelin maintenance. Upon a poorly known misfolding event, the PrP^{C} can undergo a structural change and acquire an alternative conformation named PrP^{Sc} (from scrapie) or prion. This aberrant isoform presents a high β-sheet content, becoming protease-resistant and aggregation prone, forming amyloid fibrils with the capacity to induce the same conformation on natively folded PrP^{C}, and thus, propagating and spreading through a self-templating polymerization process. Apart from protease resistance and self-propagating capacity, PrP^{Sc} is also neurotoxic, its accumulation leading to neurodegeneration. Taking into account the proteic nature of prions and their propagation mechanism, TSE can arise from three different origins: 1) in the acquired or infectious forms of disease, PrP^{Sc} is acquired from exogenous sources via ingestion of prion contaminated biological tissues or fluids or via medical procedures with prion contaminated materials. This exogenous PrP^{Sc} then initiates its propagation process at the expense of the PrP^{C} from the new host, invading the central nervous system and causing neurodegeneration. 2) In the familial or genetic TSE, mutations in the gene encoding PrP^{C} result in an enhanced misfolding capacity, eventually leading to PrP^{Sc} formation that initiates the propagation process, presenting this kind of TSE an autosomal dominant inheritance. 3) Finally, in the idiopathic or sporadic forms of TSE, supposedly a rare event of wild-type PrP^{C} misfolding occurs, in the absence of pathology-associated mutations, leading again to PrP^{Sc} formation and posterior propagation. Moreover, two remarkable phenomena, also closely related to the prion propagation mechanism, have been reported in TSE, the existence of a transmission barrier and the existence of different prion strains. The latter are defined as different PrP^{Sc} conformers that can show strikingly different biochemical and biological properties and thus, cause different disease types. Given the influence of the amino acid sequence of a protein in its conformation, it is not rare that PrP^{Sc} from distinct species or polymorphic variants can show different conformations with distinctive properties, constituting different strains. But in the case of prions, the strain phenomena occurs also for PrP^{Sc} formed by the same PrP sequence, being also possible to keep determined strain features with different sequences, as the phenomena is based on structural compatibility between the PrP^{Sc} conformation and the ability of a PrP^{C} to acquire exactly an identical structure. The requirement of structural compatibility for the propagation of a specific prion strain leads to the occurrence of the second phenomenon, the existence of a transmission barrier. When a PrP^{Sc} from one species or polymorphic variant is acquired by a new host with the same PrP^{C} sequence, it propagates without any barrier in the new host, easily inducing its conformation and thus, strain features to the PrP^{C} from the host. However, when the PrP^{C} sequence from the new host is different from the acquired PrP^{Sc} sequence, it can find difficulties to propagate at the expense of this PrP^{C}. If completely unable to propagate there is a strong transmission barrier between PrP^{Sc} and PrP^{C}, being the latter unable to adopt the same conformation. Nonetheless, this barrier can be more easily overcome in other cases, sometimes keeping strain features despite an initial delay in disease progression, and other times through the emergence of a new strain in the host likely through imperfect templating. Hence, the capacity of a determined prion strain to induce its conformation or an alternative misfolding at the expense of a different PrP^{C} sequence defines the host range of the strain. Since the exact molecular mechanisms resulting in strong or weak transmission barriers are completely unknown, the transmissibility of a prion strain to new hosts is currently unpredictable, as well as the possibility of new strains emerging upon transmission, being necessary to evaluate the existence of such barrier for each specific PrP^{Sc}-PrP^{C} pair.

Given the particular propagation mechanism of prions, the presence of natively folded or cellular PrP isoform, able to adopt a prion conformation, is essential as a conversion substrate for prion multiplication and spreading, and thus for the development of the neurodegenerative pathology. This fact has been proven in multiple ways, being the most remarkable the generation of PrP knockout transgenic mouse models that are completely resistant to prion diseases, even upon intracerebral inoculation of prions from exogenous sources, a procedure that is invariably fatal in wild-type animals. Thus, both PrP^{Sc} with its self-templating capacity, which act as a propagation nuclei or seed, and PrP^{C}, acting as misfolding reaction substrate, as well as some interaction between them are required for the development of a TSE.

In addition, the physical nature of prion misfolding and propagation process allows its replication *in vitro,* even in cell-free systems, in which the addition of minute amounts of PrP^{Sc} (seed) induce the conversion of the PrP^{C} present in the reaction substrate, further proving that PrP^{C}-PrP^{Sc} interaction is required for prion pathogenesis. Several methods have been developed for *in vitro* prion propagation, faithfully reproducing the self-templated propagation occurring in the central nervous system of diseased individuals. Among these systems, the Protein Misfolding Cyclic Amplification (PMCA) technique, based on alternate sonication and incubation cycles is one of the most successful since it keeps the strain characteristics and recapitulates transmission barriers. Further developments of this method, initially based on brain homogenates of healthy animals as PrP^{C} source or substrate, and PrP^{Sc} from brains of diseased individuals as seed, have led to the possibility of reproducing also spontaneous prion misfolding. This reaction is performed in absence of exogenous PrP^{Sc} seeds, allowing the generation of distinct strains of infectious prions *de novo.* Finally, the latest development in this regard led to the Protein Misfolding Shaking Amplification (PMSA) assay, derived from the PMCA, substituting sonication for shaking and allowing the use of recombinant PrP (rec-PrP) produced in bacteria as reaction substrate, and thus offering unprecedented possibilities to evaluate misfolding capacity of all imaginable PrP sequences and artificial variants.

The invariably fatal nature of prion diseases, as well as their transmissibility and zoonotic potential, able to cause epidemics such as that caused by transmission of bovine spongiform encephalopathy-causing prions to humans resulting in the new variant Creutzfeldt-Jakob disease has heightened the urgency to develop therapies for the TSE. Since the disease is driven by PrP^{C} to PrP^{Sc} conversion, the search of compounds able to inhibit this process of Prp^{Sc} formation is one of the obvious therapeutic approaches. Either targeting PrP^{C}, using for example molecular chaperones increasing the stability of their native conformation, or PrP^{Sc}, trying to enhance the clearance of aggregates, or through compounds able to inhibit PrP^{C}-PrP^{Sc} interactions, limiting prion propagation and spreading.

As previously noted, to date there is no available cure for prion diseases and so far just a few clinical attempts have been made to treat these diseases, or at least to provide diagnostic neuroimaging agents. A wide variety of compounds have been evaluated in *in vitro* and *in vivo* tests, but none of them has been found to be able to extend satisfactorily survival time in animal models of prion diseases. These substances include anionic molecules linked to polymers, like pentosan polysulfate (which has been used in clinical trials by intraventricular administration), sulfated glycosaminoglycans and their derivatives bearing lipophilic substituents, and polyacrylamide substituted with sulfated acetylglycosamine units. Another class of macromolecules endowed with antiprion activity is constituted by dendrimers. Anyway, macromolecules present the major drawback of absence of blood-brain barrier permeability, so most of the research on antiprion compounds concerns small molecules, including for instance cyclic tetrapyrroles (such as porphyrins and phthalocyanines); aminothiazoles and related compounds; acridines, quinolines and analogues; compounds with chaperone activity targeting the native prion protein; inhibitors of the protein-folding activity of ribosomes (PFAR); N-(aryl or heteroaryl)amides; various simple aromatic and heterocyclic compounds; and antibiotics, cholesterol-depleting agents and steroids (see Mustazza C, Sbriccoli M, Minosi P, Raggi C. Small Molecules with Anti-Prion Activity. Curr Med Chem. 2020;27(33):5446-5479. doi: 10.2174/0929867326666190927121744)

The anti-prion activity of cyclic tetrapyrroles, such as porphyrins and phthalocyanines, was described for the first time in 1998 by the group of W.S. Caughey (Caughey WS, Raymond LD, Horiuchi M, Caughey B. Inhibition of protease-resistant prion protein formation by porphyrins and phthalocyanines. Proc. Natl. Acad. Sci. USA, 1998, 95, 12117-12122. doi: 10.1073/pnas.95.21.12117; Priola, SA; Raines, A; Caughey, WS. Porphyrin and phthalocyanine antiscrapie compounds. Science 2000, 287, 1503-1506. doi: 10.1126/science.287.5457.1503; WO0009111). These compounds were shown to inhibit prion propagation *in vitro* and up to some extent *in vivo,* however the way they were administered to the animals together with the prion seed raised doubts on their potential efficacy. Moreover, despite their promising anti-prion effect, the porphyrins and phthalocyanines tested initially were not developed further as treatment for prion diseases, mainly due to administration difficulties and toxicity. Moreover, Trevitt et al (C. R TREVITT: "A systematic review of prion therapeutics in experimental models", BRAIN, vol. 129, no. 9, 1 September 2006 (2006-09-01), pages 2241-2265, XP055036135, ISSN: 0006-8950, DOI: 10.1093/brain/awl150) disclosed tetrapyrrolic compounds, but not uroporphyrins, for treating prion diseases. According to them, a variety of tetrapyrroles, including porphyrins, have already been investigated as potential anti-prion compounds and were found to decrease the levels of PrPres and prevent its propagation. TMPP-Fe3+ was included among the most promising tested compounds. Moreover, various porphin metallo-complexes, for instance Fe-TAP or Fe-TSP, were found to increase survival after scrapie infection.

Porphyrins are a group of heterocyclic organic compounds, constituted by a planar basic structure known as porphine (C₂OH₁₄N₄) formed by four modified pyrrole rings interconnected at their α carbon atoms via methine bridges (=CH-), altogether forming a closed ring named tetrapyrrol.

Many porphyrins are found in living organisms, showing a high diversity and being part of abundant cell components such as chlorophyll, B12 vitamin or the heme group (critical part of hemoglobin). In some cases, these porphyrins form complexes with metal ions, being known as metalloporphyrins, as happens with the porphyrins forming part of myoglobin and hemoglobin, complexed with an iron ion. The most representative of the latter group of natural compounds, all part of the heme biosynthetic pathway, are the uroporphyrins, coproporphyrins and penta, hexa and heptaporphyrins.

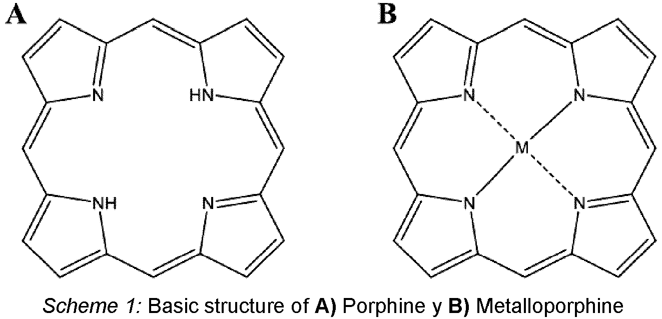

The function of porphyrins is highly variable, depending on the groups or radicals that can be bound to the pyrrole rings or the α carbon atoms keeping these groups together. A unique group of natural porphyrins is that of uroporphyrins, intermediate compounds in the biosynthesis of the heme group, synthesized in animals from glycine and succinyl-CoA through a complex multienzymatic pathway with several intermediate products such as δ -aminolevulinic acid, porphobilinogen, uroporphyrinogen, coproporphyrinogen and protoporphyrinogen, finally converted into protoporphyrin, which in complex with iron ions constitutes the heme group. Given the strict regulation of this biosynthetic pathway, almost all natural porphyrins in animals are part of hemoglobin, myoglobin or the respiration ferment. However, a small amount of some of these compounds can be found free, namely, uroporphyrins, coproporphyrins and protoporphyrins, as byproducts of the heme biosynthetic pathway.

Two large groups of porphine derivatives can be also distinguished depending on the binding sites of radicals: those with lateral groups bound directly to the pyrrole rings, and those bound to the α carbon atoms linking the pyrrole groups.

Examples of compounds from the first group:

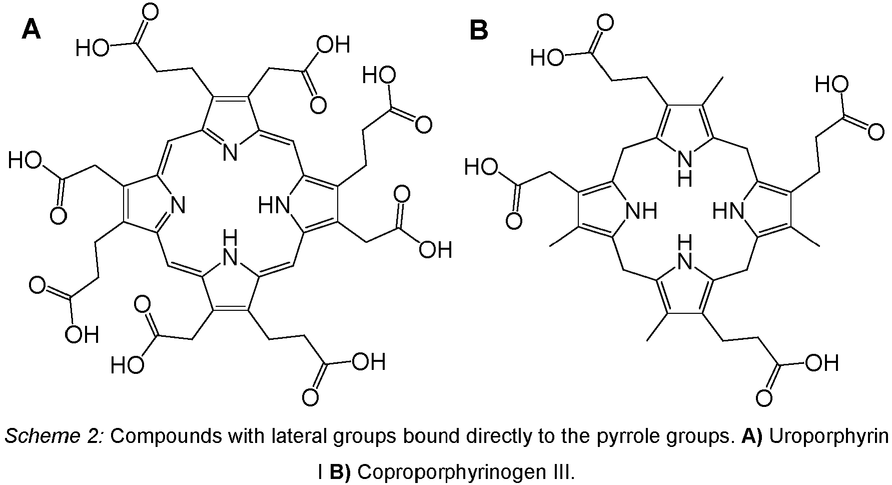

Examples of compounds from the second group:

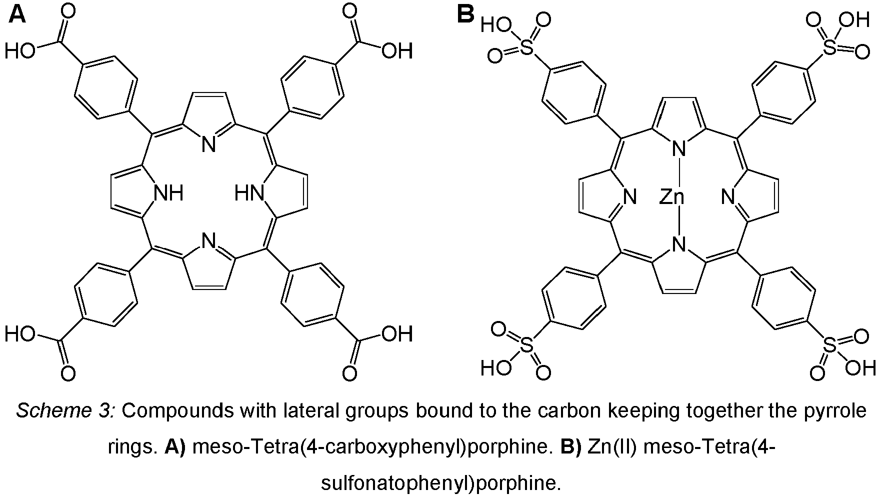

However, since the pioneering work of Caughey over 20 years ago porphyrins and phthalocyanines have not attracted much attention as potential therapeutic compounds against prion diseases but other substances have emerged as more promising candidates including ASOS (Antisense Oligonucleotides) [Raymond GJ, Zhao HT, Race B, Raymond LD, Williams K, Swayze EE, Graffam S, Le J, Caron T, Stathopoulos J, O'Keefe R, Lubke LL, Reidenbach AG, Kraus A, Schreiber SL, Mazur C, Cabin DE, Carroll JB, Minikel EV, Kordasiewicz H, Caughey B, Vallabh SM. Antisense oligonucleotides extend survival of prion-infected mice. JCI Insight. 2019 Jul 30;5(16):e131175. doi: 10.1172/jci.insight.131175] and antibody-based immunotherapy where the fully humanised prion protein monoclonal antibody PRN100 outstands as being the first drug used in human trials on CJD patients with very encouraging results (Mead S, Khalili-Shirazi A, Potter C, Mok T, Nihat A, Hyare H, Canning S, Schmidt C, Campbell T, Darwent L, Muirhead N, Ebsworth N, Hextall P, Wakeling M, Linehan J, Libri V, Williams B, Jaunmuktane Z, Brandner S, Rudge P, Collinge J. Prion protein monoclonal antibody (PRN100) therapy for Creutzfeldt-Jakob disease: evaluation of a first-in-human treatment programme. Lancet Neurol. 2022;21(4):342-354. doi: 10.1016/S1474-4422(22)00082-5).

Altogether, there are no effective treatments for prion diseases for the time being. With the exception of PRN100, clinical trials in humans have not met with success and have been hampered by the rarity of prion diseases. At present, treatment mainly focuses on providing supportive care. Examples of this type of care include:
- Medications. Some medications can be prescribed to help treat symptoms, including for instance reducing psychological symptoms with antidepressants or sedatives, providing pain relief using opiate medication or easing muscle spasms with drugs like sodium valproate and clonazepam.
- Assistance. As the disease advances, many people need help taking care of themselves and performing daily activities.
- Providing hydration and nutrients. In advanced stages of the disease, IV fluids or a feeding tube may be required.

Thus, there is still a great need of finding an effective treatment for prion diseases.

### Summary of the Invention

Described herein is a novel treatment for transmissible spongiform encephalopathies (TSE) or prion diseases, both terms being used interchangeably. In particular, we demonstrate that uroporphyrin I (UROI) is able to inhibit prion propagation *in vitro* using brain homogenates from mice to a much greater extent than TMPyP-Fe³⁺, the most effective anti-TSE porphyrin described by Caughey and collaborators (see Example 1, Figure 1). Further, the inhibitory effect of uroporphyrin I is produced in a strain-independent manner (see Example 1, Figure 2). Advantageously, UROI can also inhibit human prion propagation (see Example 2, Figure 3) and the anti-prion effect found *in vitro* was confirmed *in vivo* with transgenic mice lines genetically modified to accumulate high levels of systemic UROI, which were inoculated with different prion strains and in all the cases a delay in the disease onset was appreciated with respect to the wild-type animals (see Example 3, Figures 5 and 6). Any references in the description to methods of treatment refer to the compounds, pharmaceutical compositions and medicaments of the present invention for use in a method for treatment of the human (or animal) body by therapy.

Therefore, the present invention relates to uroporphyrin I or a pharmaceutically acceptable salt or metallo-complex thereof for use in the treatment and/or prevention a prion disease.

A further aspect is a pharmaceutical composition for use in the treatment and/or prevention of a prion disease, said composition comprising uroporphyrin I, or a pharmaceutically acceptable salt or metallo-complex thereof, and a pharmaceutically acceptable excipient.

Also, is disclosed a method for the treatment and/or prevention of a prion disease in a subject, said method comprising the administration of a therapeutically effective amount of uroporphyrin I, or a pharmaceutically acceptable salt or metallo-complex thereof to said subject. In a preferred embodiment, said subject is a human being.

In a particular embodiment of the present invention, the prion disease is selected from the group consisting of Creutzfeldt-Jakob diseases (CJD), Gerstmann-Sträussler-Scheinker syndrome (GSS), fatal familial insomnia (FFI), sporadic fatal insomnia (sFI), Variably Protease-Sensitive Prionopathy (VPSPr), kuru, scrapie, transmissible mink encephalopathy (TME), chronic wasting disease (CWD), bovine spongiform encephalopathy (BSE) and feline spongiform encephalopathy (FSE).

These aspects and preferred embodiments thereof are additionally also defined hereinafter in the detailed description and in the claims.

All the features described in this specification (including the claims, description and drawings) can be combined in any combination, with the exception of combinations of such mutually exclusive features.

### Brief Description of the Drawings

To better understand the invention, its objects and advantages, the following figures are attached to the specification in which the following is depicted:

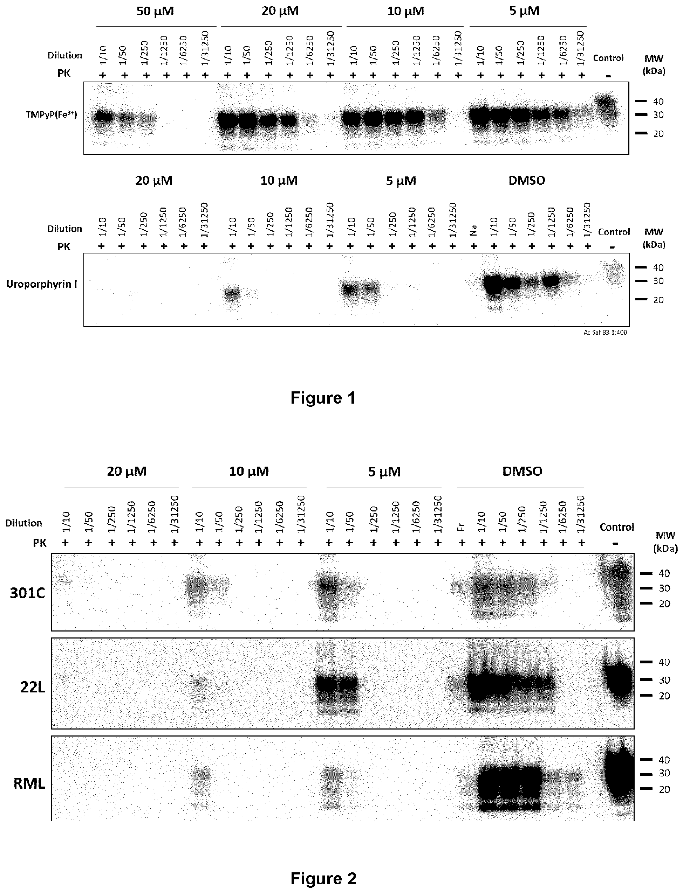

**Figure 1****. Comparative evaluation of the prion propagation inhibitory capacity *in vitro* of uroporphyrin I and TMPyP(Fe³⁺). To assess the efficacy on *in vitro* prion** propagation inhibition by UROI and TMPyP(Fe³⁺), a 24 h PMCA reaction was performed, using wild-type mouse brain homogenate as substrate and the murine prion strain RML as seed. The seed was serially diluted in the substrate (1:10, 1:50, 1:250, 1:1250, 1:6250 and 1:31250) and each compound was added to the samples at 20, 10 and 5 µM in the case of UROI and 50, 20, 10 and 5 µM in the case of TMPyP(Fe³⁺). DMSO used as diluent of both compounds was also added to a set of dilutions as positive control of prion propagation. All PMCA products were analyzed for misfolded PrP through PK-digestion, electrophoresis and Western blotting, using Saf-83 (1:400) as primary anti-PrP antibody. Both UROI and TMPyP showed prion propagation inhibitory capacity, since the highest dilution in which PrP^{Sc} was detected was lower in both cases than in the DMSO control. Nonetheless, UROI was 125 times more efficient as inhibitor, since it reduced prion propagation at the lowest concentration tested of both compounds. PK: Proteinase K. MW: Molecular weight. See Example 1.
**Figure 2****. Determination of potential prion strain specificity in the prion propagation inhibitory effect of uroporphyrin I.** To check if the prion propagation inhibitory capacity observed for UROI is strain dependent, a PMCA was performed using wild-type mouse brain homogenate as substrate and three different murine prion strains, RML, 22L and 301C, as seeds, serially diluted (1:10, 1:50, 1:250, 1:1250, 1:6250 and 1:31250) in the substrate. UROI was added to sets of serial dilutions for each strain, using three different concentrations (20, 10 and 5 µM) to check also dose-dependent effect. A set of dilutions with DMSO instead of UROI was also submitted to PMCA as positive control of prion propagation efficiency for each strain. After a single 24h PMCA round, all PMCA products were analyzed for PrP^{Sc} through PK digestion, electrophoresis and Western blotting (primary antibody Saf83 at 1:400), attending to the highest dilution in which PrP^{Sc} was detectable as a measurement of prion propagation efficiency. Compared to the highest dilutions in which PrP^{Sc} was detectable in DMSO controls, UROI showed similar inhibitory capacity for the three strains, suggesting a strain-independent mechanism of action. It reduced prion propagation 125 times at 5 µM, ~3125 times at 10 µM and almost completely at 20 µM. PK: Proteinase K. MW: Molecular weight. See Example 1.

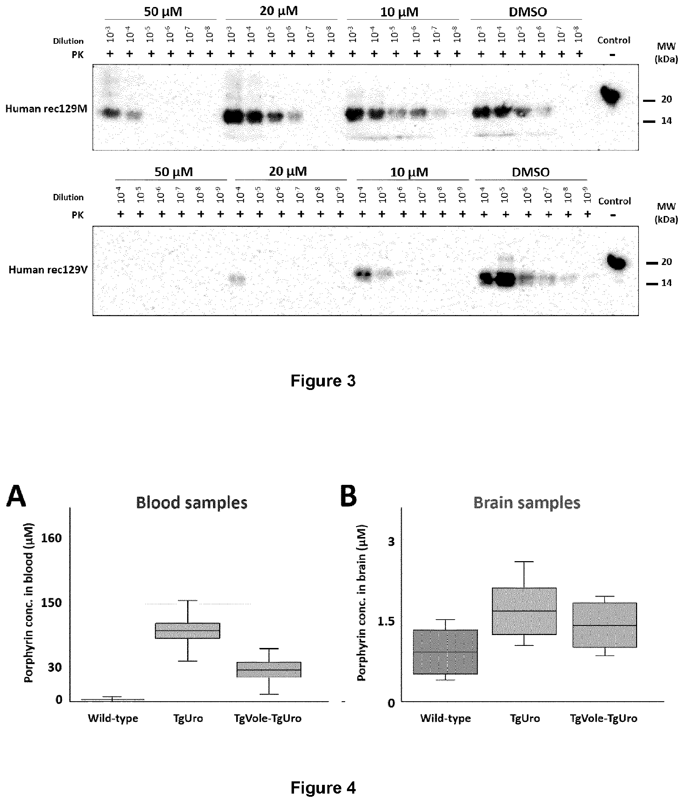

**Figure 3****. Evaluation of human prion propagation inhibitory capacity of uroporphyrin** I *in vitro.* To address whether UROI can also inhibit human prion propagation, recombinant PrP with the two main human polymorphic variants (M129 and V129) were produced in *E. coli and* complemented with PrP-knockout brain homogenate to use as PMCA substrates. As a seed, a previously generated recombinant human prion (M129) was used, serially diluted 1:10 in each substrate from 10⁻³ to 10⁻⁹. UROI was added to the dilution series at 50, 20 and 10 µM, including also controls with DMSO as positive prion propagation efficiency controls in each substrate in absence of UROI. After a 24 h PMCA reaction, all products were analyzed for PrP^{Sc} through PK digestion, electrophoresis and Western blotting (primary antibody 3F4 at 1:10,000), attending to the highest dilution in which PrP^{Sc} was detectable as a measurement of prion propagation efficiency. In the case of rec-human PrP with M129 variant, the inhibitory effect of UROI was reduced with respect to V129 variant, reaching a 5 log reduction at 50 µM, 4 logs at 20 and 2 logs at 10 µM. These results suggest that UROI could be exerting a differential effect due to possibly distinct binding affinities to M129 or V129 variants, suggesting a mechanism of action dependent on UROI-PrP^{C} binding. PK: Proteinase K. MW: Molecular weight. See Example 2.
**Figure 4****. Determination of uroporphyrin I levels in brains and blood of wild-type mice compared to TgUro and TgVole-TgUro mice to corroborate that increased levels could be responsible for prion propagation inhibition observed** *in vitro.* Wild-type mice, TgUro and TgVoleTgUro mice were culled to examine UROI levels in blood and brain, in order to correlate increased UROI levels with a potential prion propagation inhibition *in vivo.* Blood was extracted by submandibular puncture a day prior to the sacrifice of the animals, when the brains were thoroughly perfused before extraction to avoid contamination from blood derived UROI in this tissue. Both types of samples were processed for intracellular UROI extraction and UROI concentration was measured through HPLC coupled to a fluorescence detector. A) In the blood of wild-type animals, UROI levels ranged from 0 to 2 µM, whereas TgUro mice showed levels between 40 and 160 µM, with mean levels two orders of magnitude higher in these models. On the other hand, blood UROI levels in TgVole-TgUro animals ranged from 10 to 50 µM. B) Regarding UROI levels in brain, differences were reduced due to the low permeability of UROI to the blood brain barrier, ranging from 0.3-1.5 in wild-type animals and from 1.1 to 2.7 µM in TgUro or 0.8 to 1.9 µM in TgVole-TgUro although the mean value in this model was significantly higher, what could be enough to detect differences in survival time upon prion inoculation given the effectivity shown by UROI in prion propagation inhibition *in vitro.* See Example 3.

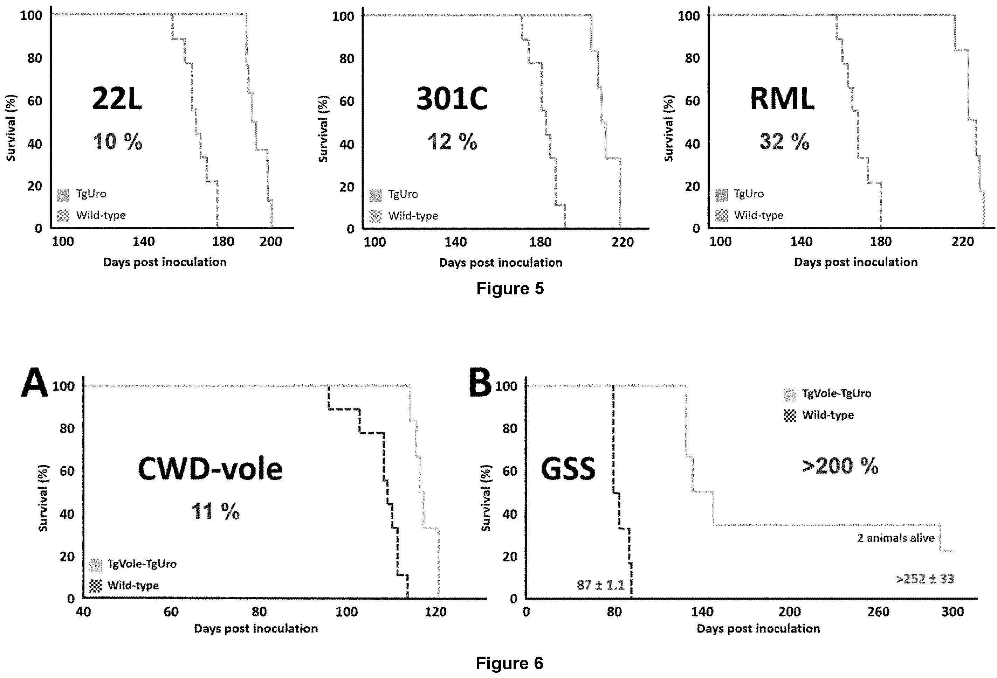

**Figure 5****. Assessment of the effectiveness of increased uroporphyrin I levels to inhibit murine prion propagation and increase survival time or delay disease onset** *in vivo.* To assess if the increased UROI levels observed in TgUro mice are sufficient to inhibit prion propagation *in vivo,* leading to an increase in survival time or delay on disease onset, validating the anti-prion effect found *in vitro,* wild-type and TgUro animals were inoculated intracerebrally with three different murine prion strains, RML, 22L and 301C. Prion infected animals were monitored daily for clinical signs of neurological disease, when they were culled and blood and brain were extracted for analysis. Survival times (equivalent to clinical disease onset time) as days post-inoculation (dpi) are represented through Kaplan-Meier survival curves, comparing survival times of wild-type and TgUro animals for each prion strain. The TgUro mice inoculated with the RML strain are those showing the highest increase in survival times with respect to wild-type animals with 30 %, whereas when inoculated with 22L and 301C survival time prolongation reached 10 and 12 % respectively, showing that in all cases, high levels of UROI are able to inhibit prion propagation *in vivo* delaying disease onset. See Example 3.
Figure 6. Assessment of the effectiveness of increased uroporphyrin I levels to inhibit prion propagation from different species and increase survival time or delay disease onset *in vivo.* To ascertain if increased UROI levels in brain and blood can inhibit *in vivo* the propagation of prions from other species apart from mouse, the TgVole-TgUro hybrid model, expressing bank vole PrP^{C} instead of murine PrP^{C} was chosen. Since they are susceptible to infection with prions from different species (including human prions) and show increased levels of UROI, albeit lower than those found in TgUro, they were: A) inoculated intracerebrally with Vole-CWD prion strain, bank vole prions derived from serial inoculations of the chronic wasting disease prions from cervids, and thus completely different from the murine prions previously used. This prion strain inoculated in bank vole, leads, in addition, to the fastest developing prion disease reported in any animal model, being suitable to test the inhibitory effect of UROI *in vivo.* B) inoculated intraperitoneally with a human prion strain causing Gerstmann-Sträussler-Scheinker syndrome (GSS). The brain homogenate containing human GSS prions was inoculated intraperitoneally in this case, given that this way of administration could mimic better the slower disease progression likely occurring in humans, prions requiring a neuroinvasion process to establish an effective infection. Prion infected animals were monitored daily for clinical signs of neurological disease, when they were culled and blood and brain were extracted for analysis. Survival times (equivalent to clinical disease onset time) as days post-inoculation (dpi) are represented through Kaplan-Meier survival curves, comparing survival times of TgVole and TgVole-TgUro hybrid animals. **A)** In the case of the animals inoculated with Vole-CWD, an 11% increase in survival time was observed in comparison to TgVole animals, with UROI levels comparable to those of a wild-type mice. Thus, the effectiveness of increased UROI levels on delaying prion disease onset was confirmed, as well as the strain and species independent inhibitory effect. **B)** In the case of the animals inoculated with human prions (GSS), an unprecedented > 200 % increase in survival time was observed in comparison to TgVole animals, being 2 of the inoculated animals free of neurological signs at present. Therefore, the effectiveness of increased UROI levels on delaying human prion disease onset was demonstrated, the higher effectiveness likely explained by the increased systemic UROI levels that may interfere with peripheral prion propagation and neuroinvasion. See Example 3.

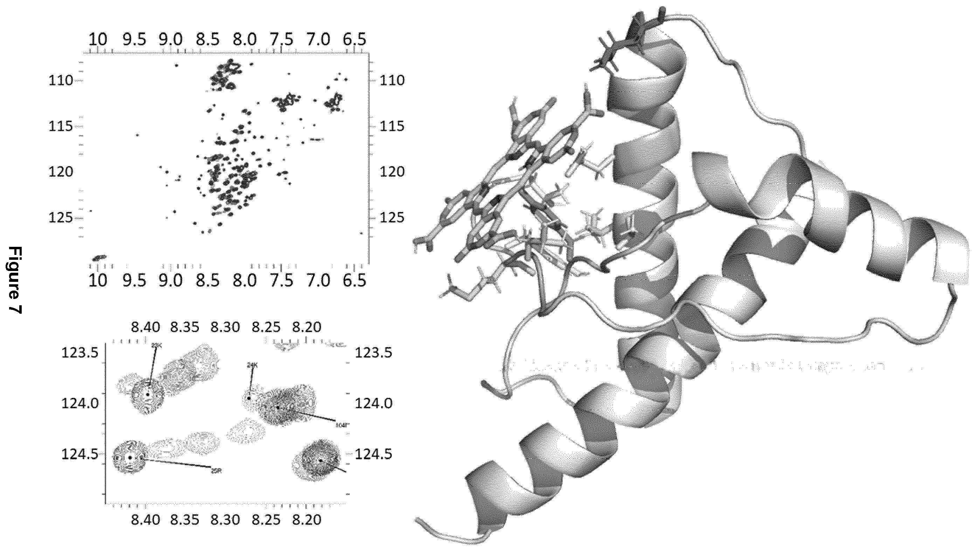

**Figure 7****. Determination of the interaction site and affinity of uroporphyrin I with human PrP M129 through Nuclear Magnetic Resonance.** To assess if UROI could exert its prion propagation inhibitory effect through PrP^{C} binding and if so, determine affinity and interaction sites, recombinant human PrP with M129 variant was produced in *E.coli,* purified and mixed with UROI at distinct concentrations, getting the Heteronuclear Single Quantum Coherence (HSQC) spectra in which chemical shifts of interacting residues can be detected. Signals corresponding to each UROI:PrP ratio are indicated in the figure by the following grey code: intense 1:0, high 1:0.2, medium 1:0.5 and low 1:0.75. Zooming into a specific area of the spectrum, the region where chemical shift is more notable is shown, indicating preferential interaction of those residues of the protein with UROI (namely residues 23K, 24K, 25R and 104K of the human PrP). A three-dimensional model of the PrP-UROI interaction is also shown. See Example 4.

### Detailed Description of the Invention

In the year 2000 the group of W. S. Caughey (Science 2000, 287, 1503-1506) reported the string anti-prion activity of a phthalocyanine (PcTS) and two distinct metalloporphyrins (DPG₂-Fe³⁺ y TMPyP-Fe³⁺), tested *in vitro* and *in vivo.* PcTS was the most effective compound. Despite a great delay observed in the time to death, the simultaneous administration of the compounds and the inoculation of prions in the same place, raising doubts about real effectiveness, and the toxicity of the compounds in therapeutic doses, impeded their development as a feasible therapeutic strategy at the time. Moreover, antibodies and their derivatives are on the current list of most prominent candidates for the treatment of prion diseases and research efforts are now being focused on this direction.

However, grounded on the Caughey's work, we explored the potential anti-prion effect of other natural porphyrins, likely less toxic than those previously tested. Uroporphyrin I (UROI), a byproduct of the heme biosynthetic route that is found in excess in some forms of porphyria, was selected as an anti-prion candidate, due to the availability of animal models naturally presenting high levels of this compound as well as its potent anti-prion activity *in vitro.* Thus, the capacity to inhibit prion propagation for the porphyrin selected for the development of a therapeutic strategy for TSE was evaluated first in cell-free prion propagation systems, PMCA and PMSA, and in different animal models. In both cases, UROI showed an extraordinary capacity to block or reduce prion propagation. Moreover, unexpectedly, the inhibitory capacity of the UROI was found to be 125 times more pronounced than that of TMPyP-Fe³⁺, the most active porphyrin proposed by Caughey.

There are four isomers of uroporphyrin, denoted I, II, III and IV by Fischer to show the four ways in which the acetic acid groups (Ac) and propionic acid groups (Pr) are arranged around the eight β-positions of the porphyrin macrocycle. The chemical structure is depicted below in Scheme 4:

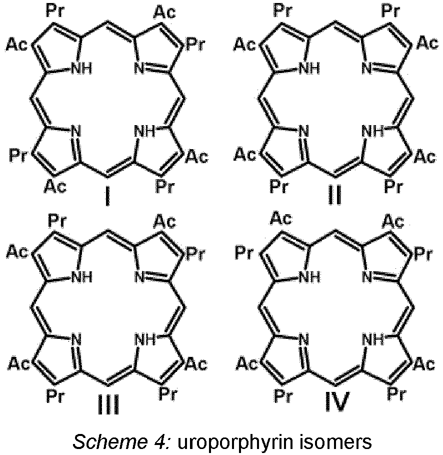

The present invention relates to uroporphyrin I (UROI) or a pharmaceutically acceptable salt or metallo-complex thereof.

By "pharmaceutically acceptable" is meant herein a material that is not biologically or otherwise undesirable, i. e., the material may be incorporated into a pharmaceutical composition administered to a patient without causing any undesirable biological effects or interacting in a deleterious manner with any of the other components of the composition in which it is contained. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The invention also contemplates "salts" of uroporphyrin I. By way of illustration, said salts can be acid addition salts, base addition salts or metal salts. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, p-toluenesulfonate, trifluoroacetate, camphorsulfonate, etc. Illustrative examples of base addition salts include inorganic base salts such as, for example, ammonium salts and organic base salts such as, for example, ethylenediamine, ethanolamine, N,N-dialkylenethanolamine, triethanolamine, glutamine, amino acid basic salts, etc. Illustrative examples of metal salts include, for example, sodium, potassium, calcium, magnesium, aluminum and lithium salts.

According to a particular embodiment, the salt of uroporphyrin is Uroporphyrin I dihydrochloride (CAS 68929-06-6).

Metallo-complexes of uroporphyrin I are formed by replacing the central hydrogens of the porphine ring with a metal, metalloid, or a metal compound. The center nitrogens of uroporphyrin I may bond to and form complexes with virtually all metals and metalloids. At least one of the four nitrogens at the center of the tetrapyrrole macrocycle may participate in complex formation. The metal or metalloid may be in the form of an ion, such as Fe³⁺, or a compound, such as VO. Metals or metalloids that form uroporphyrin complexes include Li, Na, Mg, B, Al, Si, P, K, Ca, Sc, Ti, V, Cr, Mn, Fe, Co, Ni, Cu, Zn, Ga, Ge, As, Rb, Sr, Y, Zr, Nb, Mo, Tc, Ru, Rh, Pd, Ag, Cd, In, Sn, Sb, Cs, Ba, La, Hf, Ta, W, Re, Os, Ir, Pt, Au, Hg, Tl, Pb, Bi, Th, and the lanthanides. Included are complexes wherein the metal or metalloid is of a specific oxidation state. For example, uroporphyrin I may form complexes with both Co²⁺ and Co³⁺. Also included are uroporphyrin I complexes wherein the metal, metalloid, or metal compound has additional ligands. As used in this specification, the prefix "metallo" includes complexes formed with metalloids, unless specifically stated otherwise.

Typical metals which can be incorporated into the uroporphyrin I structure are Iron (Fe), Cobalt (Co), Gallium (Ga), Tin (Sn), Zinc (Zn), Chromium (Cr), Magnesium (Mg), and the various elements of the lanthanide series.

There has been to the inventors' knowledge no disclosure of the utility of any uroporphyrin (or a pharmaceutically acceptable salt or metallo-complex thereof) in the treatment of a prion disease.

The present invention relates to the use of uroporphyrin I or a pharmaceutically acceptable salt or metallo-complex thereof for the manufacture of a medicament or pharmaceutical composition for the treatment and/or prevention of a prion disease.

As used herein, the term "prion diseases" refers to transmissible spongiform encephalopathies. This group of neurologic diseases affects humans and many species of animals causing a "sponge-like" degeneration of brain tissue. Among other unique features, all of these diseases are associated with the accumulation of an abnormal form of the prion protein in nerve cells that eventually leads to the death of the host. While prion diseases can all be transmitted from one host to another, it remains contentious as to whether a virus-like infectious agent or the abnormal prion protein itself, the prion, causes the conversion of normal to abnormal protein.

Probably most mammalian species develop prion diseases. Specific examples for (non-human) animals include:
- Scrapie: sheep, goat
- TME (transmissible mink encephalopathy): mink
- CWD (chronic wasting disease): mule deer, deer, elk
- BSE (bovine spongiform encephalopathy): cows, cattle
- FSE (feline spongiform encephalopathy): cats and other felines

Humans are also susceptible to several prion diseases. Examples are:
- CJD (Creutzfeldt-Jacob Disease)
- GSS (Gerstmann-Sträussler-Scheinker syndrome)
- FFI (Fatal familial insomnia)
- sFl (sporadic fatal insomnia)
- Variably Protease-Sensitive Prionopathy (VPSPr)
- Kuru

More particularly, the human prion diseases include kuru, sporadic Creutzfeldt-Jakob disease (sCJD), familial CJD (fCJD), iatrogenic CJD (iCJD), Gerstmann-Sträussler-Scheinker (GSS) disease, fatal familial insomnia (FFI), sporadic fatal insomnia (sFI), variably protease-sensitive prionopathy (VPSPr), and, more recently, new variant CJD (nvCJD or vCJD). In addition to these human diseases, prion-related diseases, have been recognized in several animal hosts. Scrapie is a naturally occurring disease of sheep and goats that causes ataxia, behavioral changes, and a severe pruritus that leads to scraping behavior, from which the disease was named. Additional prion diseases in animals include transmissible mink encephalopathy (TME), chronic wasting disease (CWD) of deer and elk, feline spongiform encephalopathy (FSE), and bovine spongiform encephalopathy (BSE), among others.

According to the present invention, any of the abovementioned prion diseases may be treated and/or prevented with uroporphyrin I (UROI) or a pharmaceutically acceptable salt or metallo-complex thereof.

In a more particular embodiment, the prion disease is selected from the group consisting of Creutzfeldt-Jakob diseases (CJD), fatal familial insomnia (FFI), sporadic fatal insomnia (sFI), variably protease-sensitive prionopathy (VPSPr), Gerstmann-Straussler-Scheinker syndrome (GSS), scrapie, bovine spongiform encephalopathy (BSE) and chronic wasting disease (CWD).

Examples of pharmaceutical compositions include any solid composition (e.g. tablets, pills, capsules, granules) or liquid composition (e.g. solutions, suspensions lotions or emulsions).

The compositions can for example be administered orally, topically, dermally, nasally, intravenously, intramuscularly, intraperitoneally, intracerobrospinally, intracranially, intraspinally, subcutaneously, intraarticularly, intrasynovialy, or intrathecaly. Other forms of administration are not excluded. In a particular embodiment, the compositions are administered intracerobrospinally or intracranially.

The respective composition may - depending on its route of administration - also contain one or more excipients known to those skilled in the art. The composition or medicament according to the present invention may be produced according to standard procedures known to those skilled in the art.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong in vivo drug actions, decrease drug metabolism, and reduce drug toxicity. Carriers are also used in designs to increase the effectiveness of drug delivery to the target sites of pharmacological actions (U.S. National Library of Medicine. National Institutes of Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

Also is disclosed a method of treatment of a patient suffering a prion disease, or likely to suffer a prion disease, which comprises administering to the patient in need of such a treatment or prophylaxis an effective amount of uroporphyrin or a pharmaceutically acceptable salt or metallo-complex thereof.

The terms "subject", "patient" or "individual" preferably refer to mammals, including humans and other mammal animals (such as sheep, goat, mink, mule deer, deer, elk, cow and other cattle, cat and other felines), especially humans or ruminants. Ruminants are, for instance, mule deer, elk, cow, cattle, sheep, goat, deer, or buffalo. Minks are an example for mammals, which do not belong to the suborder Rumiantia.

As used herein, the terms "treat", "treating" and "treatment" include in general the eradication, removal, reversion, alleviation, modification, or control of the prion disease after its onset.

As used herein, the terms "prevention", "preventing", "preventive", "prevent" and "prophylaxis" refer to the capacity of a given substance, composition or medicament to avoid, minimize or difficult the onset or development of the prion disease before its onset.

By an "effective" amount or a "therapeutically effective amount" of a drug or pharmacologically active agent is meant a nontoxic but sufficient amount of the drug or agent to provide the desired effect. In the therapy of the present invention, an "effective amount" of uroporphyrin I or a pharmaceutically acceptable salt or metallo-complex thereof is the amount of that compound that is effective to provide the desired effect. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation.

Uroporphyrin I or a pharmaceutically acceptable salt or metallo-complex thereof can be used together with other additional useful drugs in the prevention and/or treatment of a prion disease. Said additional drugs can form part of the same pharmaceutical composition or, alternatively, can be provided in the form of a separated composition for their simultaneous or sequential administration to that of the pharmaceutical composition comprising uroporphyrin I or a pharmaceutically acceptable salt or metallo-complex thereof.

According to a particular embodiment of the present invention, uroporphyrin I or a pharmaceutically acceptable salt or metallo-complex thereof is intended to inhibit, block or reduce prion propagation.

According to another embodiment of the present invention, uroporphyrin I or a pharmaceutically acceptable salt or metallo-complex thereof is intended to increase survival time and/or delay disease onset.

According to another embodiment of the present invention, uroporphyrin I or a pharmaceutically acceptable salt or metallo-complex thereof is intended to inhibit, block or reduce spongiform changes in the brain.

According to another embodiment of the present invention, uroporphyrin I or a pharmaceutically acceptable salt or metallo-complex thereof is intended to inhibit, block or reduce intense vacuolation due to extensive neuronal death.

According to another embodiment of the present invention, uroporphyrin I or a pharmaceutically acceptable salt or metallo-complex thereof is intended to inhibit, block or reduce the accumulation of amyloid plaques or deposits in the central nervous system.

According to another embodiment of the present invention, uroporphyrin I or a pharmaceutically acceptable salt or metallo-complex thereof is intended to inhibit, block or reduce the conversion of PrP^{C} to PrP^{Sc}.

The following examples are provided as supporting evidence for the invention since they demonstrate that UROI showed an extraordinary capacity to block or reduce prion propagation both *in vitro* and *in vivo.* The following examples are merely illustrative of certain embodiments of the invention and cannot be considered as restricting it in any way.

### Examples

### Example 1. Evaluation of the capacity of the Uroporphyrin I to inhibit prion propagation in vitro by brain homogenate-based PMCA

To assess if the Uroporphyrin I (UROI) could be a good inhibitor of prion propagation, its capacity to reduce or block prion propagation was tested by PMCA, a cell-free prion amplification system based on brain homogenates from healthy animals as substrate (PrP^{C} source) and brain homogenates from animals infected with prions at terminal stage (as source of PrP^{Sc}) as seeds. Specifically, 10 % (w/V) homogenates of perfused brains from C57BI6 wild-type mice, homogenized in Conversion Buffer, were used as reaction substrate, and to determine potential strain specific effects of the compound, three different murine prion strains (RML, 22L and 301C) as seeds. The latter coming from 10 % brain homogenates of mice previously inoculated with each prion strain and sacrificed after the onset of obvious neurological signs of disease.

First, to compare the inhibitory capacity of the UROI to that of the TMPyP-Fe³⁺, the most effective porphyrin described by Caughey and collaborators (Science 2000, 287, 1503-1506), they were added at different concentrations to the PMCA substrate (5, 10, 20 and 50 µM). As both compounds were diluted in DMSO, this solvent was added in the same volume to the control reaction, with no inhibitors. Then, brain homogenate containing RML prion strain was serially diluted in the reaction substrate, performing dilutions 1:10, 1:50, 1:250, 1:1250, 1:16250 and 1:31250 (seed : substrate) for each compound concentration and the DMSO control, in order to quantify the inhibition capacity. All samples were afterwards submitted to a 24 h PMCA round, in a horn cup sonicator (Misonix Q-700, Qsonica) programmed for alternate cycles of 30 min of incubation and 20 s of sonication at 80 % amplitude, keeping the temperature constant at 38 °C throughout the process with a circulating water bath. Prion propagation or amplification was then monitored through proteinase K (PK) digestion of all the PMCA products, electrophoresis and Western blotting for the detection of misfolded, PK-resistant PrP. The highest dilution of the seed in which this PrP^{Sc} is detected after PMCA provides the measurement of prion propagation efficiency, and its reduction compared to the limit dilution of the DMSO control, the measurement of the inhibitory efficacy of the compounds tested.

Both UROI and TMPyP showed prion propagation inhibitory capacity, since the highest dilution in which PrP^{Sc} was detected was lower in both cases than in the DMSO control. Nonetheless, UROI was 125 times more efficient as inhibitor, since it reduced prion propagation at the lowest concentration tested of both compounds. See Figure 1.

In a similar way, the strain specificity of UROI inhibitory capacity was also evaluated using three different murine prion strains as seeds. With the same PMCA substrate prepared from perfused brains of C57BI6 mice, the inhibitory effect of UROI at 20, 10 and 5 µM was tested, performing serial dilutions (1:10, 1:50, 1:250, 1:1250, 1:16250 and 1:31250) of the three prion seeds for each compound concentration. Again, DMSO (as vehicle for UROI solution) was added to the propagation controls in order to determine the propagation efficiency of each prion strain in absence of UROI. All samples were submitted to a 24 h PMCA round in the same conditions as before and prion propagation was monitored by PK digestion electrophoresis and Western blotting, attending to the highest dilution in which PrP^{Sc} is detectable after PMCA for each strain in comparison to the DMSO controls.

As shown in Figure 2, compared to the highest dilutions in which PrP^{Sc} was detectable in DMSO controls, UROI showed similar inhibitory capacity for the three strains, suggesting a strain-independent mechanism of action. It reduced prion propagation 125 times at 5 µM, ~3125 times at 10 µM and almost completely at 20 µM.

### Example 2. Evaluation of the capacity of the Uroporphyrin I to inhibit human prion propagation in vitro by recombinant PrP-based PMCA

Given the limitations for testing the inhibitory effect of compounds *in vitro* using brain homogenates from human beings, the assessment of human prion-specific effect of UROI was performed using human recombinant PrP (rec-PrP) produced in *E. coli* as reaction substrate, as well as, a recombinant human prion previously generated in the laboratory as a seed. For that, human recombinant PrP containing the two main polymorphic variants (M129 and V129) were expressed in bacteria and purified by immobilized metal affinity chromatography (IMAC). After dialyzing the proteins to obtain natively folded soluble proteins, they were mixed with 10 % (w/V) perfused brain homogenates from PrP knockout transgenic mice in order to provide brain cofactors promoting prion propagation *in vitro.* Different concentrations of UROI (50, 20 and 10 µM) were added to these substrates composed by brain homogenate complemented human rec-PrP M129 and V129 and DMSO was added to their respective propagation controls. The recombinant prion seeds were then serially diluted 1:10 (from 10⁻¹ to 10⁻⁹) in each substrate for each UROI concentration and the DMSO control. All samples were afterwards submitted to a 24 h PMCA round, in a horn cup sonicator (Misonix Q-700, Qsonica) programmed for alternate cycles of 30 min of incubation and 20 s of sonication at 80 % amplitude, keeping the temperature constant at 38 °C throughout the process with a circulating water bath. Prion propagation was once again monitored by PK digestion, electrophoresis and Western blotting of all PMCA products, attending to the highest dilution in which misfolded protease resistant rec-PrP can be detected in each case.

In the case of rec-human PrP with M129 variant, the inhibitory effect of UROI was reduced with respect to V129 variant, reaching a 5 log reduction at 50 µM, 4 logs at 20 and 2 logs at 10 µM. These results suggest that UROI could be exerting a differential effect due to possibly distinct binding affinities to M129 or V129 variants, suggesting a mechanism of action dependent on UROI-PrP^{C} binding. See Figure 3.

### Example 3. Mouse models used for the assessment of prion propagation inhibitory capacity of Uroporphyrin I in vivo

Two different mouse models were used to evaluate the efficacy of Uroporphyrin I to block prion propagation *in vivo* and assess whether this could increase survival times in prion-infected animals. Due to the low solubility of UROI in aqueous solutions and the difficulties this posed to achieve high levels of circulating UROI concentrations for long periods of time in wild-type mice, an animal model naturally producing high amounts of this porphyrin were used as a proof-of-concept. This transgenic mouse line named TgUro, was genetically modified to introduce a deleterious mutation in its Uroporpyhrin III synthase (UROSIII) enzyme, leading to the dysregulation of the heme biosynthetic pathway and the accumulation of high levels of systemic UROI. Thus, this model, bearing endogenous mouse PrP^{C}, reproduces a form of porphyria, showing red-colored urine due to excretion of large amounts of porphyrins, red-colored bones and teeth, and skin photosensitivity among others, although if protected from intense UV exposure, they present a similar lifespan to a wild-type mouse. As they are the same as wild-type mice in terms of PrP^{C} expression, they should be as susceptible to murine prion infection if the UROI is not able to inhibit prion propagation *in vivo.*

As a mouse model expressing endogenous mouse PrP^{C}, the TgUro is susceptible to a limited range of prions, namely murine prions, impeding efficacy assessment of prion strains from other species. In order to overcome this limitation and evaluate the efficacy of UROI to inhibit propagation from distinct prion strains from multiple species in vivo, a second mouse model was developed. For that, transgenic mice expressing bank vole *(Myodes glareolus)* PrP^{C} instead of their endogenous mouse PrP^{C} were used, named TgVole. Bank voles, are known in the prion research filed as the universal acceptors of prions, since due to their particular PrP^{C} sequence these rodents are highly susceptible to many different prion strains from distinct mammals, including human prion strains. To achieve high systemic concentrations of UROI continuously in TgVole, they were crossbred with TgUro mice, obtaining a hybrid mice line expressing bank vole PrP^{C} and presenting high levels of UROI in blood due to the same UROSIII enzyme deficiency. This line was name as TgVole-TgUro. Apart from showing enhanced susceptibility to prion infection, this hybrid mice showed slightly lower levels of circulating UROI, posing a greater challenge to demonstrate effectiveness of UROI inhibition of prion propagation *in vivo* than the TgUro model.

### Monitoring uroporphyrin I levels in blood and brain of wild type, TgUro and TgVole-TgUro animal models through HPLC

Since most of the UROI is found in the cytosol of cells, especially in red blood cells in blood, its extraction is required for the precise measurement of UROI levels.

For the analysis of UROI levels in brain, to avoid contamination of the UROI found in blood which is orders of magnitude higher, brains were exhaustively perfused to eliminate the blood prior to extraction. These samples were then thoroughly homogenized in a 6 M HCl solution and sonicated afterwards to achieve complete lysis of cells. After incubating samples at 37 °C for 30 min they were centrifuged to eliminate all cell debris and supernatant, containing soluble UROI were filtered through membranes with 0.22 µm pores as many times as necessary to obtain a clarified flow-through. After processing, they were stored at -20 °C until measurement by High Performance Liquid Chromatography (HPLC). Extraction of UROI from blood samples was done in a similar way, diluting blood samples 1:10 in a 6 M HCl solution and sonicating them after vigorous homogenization to ensure complete lysis of cells. Again, after incubating samples at 37 °C for 30 min they were centrifuged to eliminate all cell debris and supernatant, containing soluble UROI were filtered through membranes with 0.22 µm pores as many times as necessary to obtain a clarified flow-through. After processing, they were stored at -20 °C until measurement by the same HPLC method. In all cases, triplicates of each sample were processed.

UROI detection was performed by HPLC (ALLIANCE 2695, Waters) coupled to a fluorescence detector (Waters 474 Scanning Fluorescence Detector), taking advantage of the intrinsic fluorescence of UROI. Samples were loaded in a KROMASIL 100 C18 250 x 4.6 mm chromatography column (Teknokroma, Thermo Scientific), using a gradient of two solvents as mobile phase (solution A: ammonium acetate 0.1 M at pH5.16, and solution B: acetonitrile at 100 %). 5 to 20 µl of each sample were injected into the column and measurement performed at 405 nm of emission and 610 nm of excitation, determining UROI levels based on a standard curve previously performed with pure UROI.

Wild-type mice, TgUro and TgVoleTgUro mice were culled to examine UROI levels in blood and brain, in order to correlate increased UROI levels with a potential prion propagation inhibition *in vivo.* Blood was extracted by submandibular puncture a day prior to the sacrifice of the animals, when the brains were thoroughly perfused before extraction to avoid contamination from blood derived UROI in this tissue. Both types of samples were processed for intracellular UROI extraction and UROI concentration was measured through HPLC coupled to a fluorescence detector. In the blood of wild-type animals, UROI levels ranged from 0 to 2 µM, whereas TgUro mice showed levels between 40 and 160 µM, with mean levels two orders of magnitude higher in these models. On the other hand, blood UROI levels in TgVole-TgUro animals ranged from 10 to 50 µM (see Figure 4A). **B)** Regarding UROI levels in brain, differences were reduced due to the low permeability of UROI to the blood brain barrier, ranging from 0.3-1.5 in wild-type animals and from 1.1 to 2.7 µM in TgUro or 0.8 to 1.9 µM in TgVole-TgUro although the mean value in this model was significantly higher, what could be enough to detect differences in survival time upon prion inoculation given the effectivity shown by UROI in prion propagation inhibition *in vitro* (see Figure 4B).

### Bioassays to evaluate effectiveness of uroporphyrin I inhibition of prion propagation in vivo

Groups of 5 to 8 TgUro or the TgVole-TgUro transgenic mice were used for intracerebral prion inoculation, using wild-type mice and TgVole animals as controls, respectively. Briefly, mice were inoculated intracerebrally with 1% brain homogenates from individuals previously inoculated with each prion strain and sacrificed after the onset of obvious neurological signs of disease. Specifically, TgUro and wild-type mice were inoculated with three different murine prions (RML, 22L and 301C) to assess potential strain specific effects of UROI. Where TgVole-TgUro hybrids and the respective TgVole controls were inoculated in the same way with CWD-vole prions and human Gerstmann-Sträussler-Scheinker syndrome-causing prions. The first inoculum, was originated through serial infections of bank voles with deer prions (the strain causing Chronic Wasting Disease in cervids), that resulted in the CWD-vole strain. This prion was chosen for being one of the fastest and thus, most aggressive prion strains ever described in experimental models, causing neurological signs as early as 100 days post-inoculation (dpi) in TgVole, while usually incubation times in rodent prion diseases exceed 150 dpi. In addition, the hybrid model and controls were also inoculated intraperitoneally with 10 % brain homogenate from a deceased GSS patient, taking advantage of the susceptibility of this model to human prions. Intraperitoneal inoculation was selected in this case as a closer model to a natural prion disease occurring in humans, with longer incubation periods, a potential neuroinvasion process (transport of prions from periphery to brain), and slower disease progression.

All prion-inoculated animals, housed in BSL-3 level animal facility, were monitored daily for neurological signs including kyphosis, gait abnormalities, altered coat state, depressed mental state, flattened back, eye discharge, hyperactivity, loss of body condition and incontinence, and culled when at least three of the described signs were observed simultaneously, as indicative of a neurodegenerative process. Brains from sacrificed animals were harvested, dividing both hemispheres to fix one half in formalin for anatomopathological examination and freezing the other half for biochemical studies to confirm prion infection and check for any disease modifying effect. The parameter used to determine effectiveness of UROI inhibitory capacity, was the survival time of the animals, expressed as days post-inoculation. Blood was also extracted at the terminal stage to determine UROI levels. Additionally, non-prion infected wild-type mice, TgUro and hybrid animals of different ages were used to determine UROI levels in blood and brain as detailed in the next section.

The TgUro mice inoculated with the RML strain are those showing the highest increase in survival times with respect to wild-type animals with 30 %, whereas when inoculated with 22L and 301C survival time prolongation reached 10 and 12 % respectively, showing that in all cases, high levels of UROI are able to inhibit prion propagation *in vivo* delaying disease onset (see Figure 5).

To ascertain if increased UROI levels in brain and blood can inhibit *in vivo* the propagation of prions from other species apart from mouse, the TgVole-TgUro hybrid model, expressing bank vole PrP^{C} instead of murine PrP^{C} was chosen (see Figure 6). Since they are susceptible to infection with prions from different species (including human prions) and show increased levels of UROI, albeit lower than those found in TgUro, they were: **A)** inoculated intracerebrally with Vole-CWD prion strain, bank vole prions derived from serial inoculations of the chronic wasting disease prions from cervids, and thus completely different from the murine prions previously used. This prion strain inoculated in bank vole, leads, in addition, to the fastest developing prion disease reported in any animal model, being suitable to test the inhibitory effect of UROI *in vivo.* **B)** inoculated intraperitoneally with a human prion strain causing Gerstmann-Sträussler-Scheinker syndrome (GSS). The brain homogenate containing human GSS prions was inoculated intraperitoneally in this case, given that this way of administration could mimic better the slower disease progression likely occurring in humans, prions requiring a neuroinvasion process to establish an effective infection. Prion infected animals were monitored daily for clinical signs of neurological disease, when they were culled and blood and brain were extracted for analysis. Survival times (equivalent to clinical disease onset time) as days post-inoculation (dpi) are represented through Kaplan-Meier survival curves, comparing survival times of TgVole and TgVole-TgUro hybrid animals. **A)** In the case of the animals inoculated with Vole-CWD, an 11% increase in survival time was observed in comparison to TgVole animals, with UROI levels comparable to those of a wild-type mice. Thus, the effectiveness of increased UROI levels on delaying prion disease onset was confirmed, as well as the strain and species independent inhibitory effect. **B)** In the case of the animals inoculated with human prions (GSS), an unprecedented > 200 % increase in survival time was observed in comparison to TgVole animals, being 2 of the inoculated animals free of neurological signs at present. Therefore, the effectiveness of increased UROI levels on delaying human prion disease onset was demonstrated, the higher effectiveness likely explained by the increased systemic UROI levels that may interfere with peripheral prion propagation and neuroinvasion.

### Example 4. Characterization of the potential mechanism of action of uroporphyrin I as a prion propagation inhibitor by NMR

In order to determine the potential mechanism of action by which UROI inhibits prion propagation, its ability to bind natively folded PrP was addressed. A high binding affinity would point towards UROI acting as a chemical chaperone, either stabilizing the PrP^{C} conformation or impeding PrP^{C}-PrP^{Sc} interaction through the blocking of relevant interaction sites. Nuclear Magnetic Resonance (NMR) was the technique chosen for that purpose.

N¹⁵ labeled human recombinant PrP (M129 polymorphic variant), produced in *E. coli,* purified through IMAC and dialyzed for a correct native folding was mixed with distinct amounts of UROI solution (stock at 2 mM) for titration and determination of binding affinity. A Bruker Advance III de 800 MHz RMN spectrometer was used for all titration experiments, with a 298 K cryoprobe. The following pulse program, fhsqcf3gpph [fast-HSQC, *phase-sentitive* ge 2D 1 H-15N HSQC with *watergate* (3-9-19)] was used to obtain the HSQC spectra, that were afterwards processed with *TopSpin* 4.0.6. The assignation was done using CCPNMR and taking as reference the chemical shifts from *Biological Magnetic Resonance Data Bank* (BMRB), accession number 4 402. The chemical shift analysis was completed with in-house made scripts in MatLab, automatically determining the potential UROI-PrP interaction sites. All the information was used to calculate the *in silico* molecular model of the interaction, using the CHASSYSDOCK platform (includes AutoDock VINA, Autodock 4.2 y AutoDock Tools). The structure of PrP from which the model was developed is that with PDB code 1QLZ.

To assess if UROI could exert its prion propagation inhibitory effect through PrP^{C} binding and if so, determine affinity and interaction sites, recombinant human PrP with M129 variant was produced in *E.coli,* purified and mixed with UROI at distinct concentrations, getting the Heteronuclear Single Quantum Coherence (HSQC) spectra in which chemical shifts of interacting residues can be detected. Signals corresponding to each UROI:PrP ratio are indicated in the figure by the following grey code: intense 1:0, high 1:0.2, medium 1:0.5 and low 1:0.75. Zooming into a specific area of the spectrum, the region where chemical shift is more notable is shown, indicating preferential interaction of those residues of the protein with UROI (namely residues 23K, 24K, 25R and 104K of the human PrP). A three-dimensional model of the PrP-UROI interaction is also shown in Figure 7.

## Claims

1. Uroporphyrin I (UROI) or a pharmaceutically acceptable salt or metallo-complex thereof for use in the treatment and/or prevention a prion disease.

2. Uroporphyrin I or a pharmaceutically acceptable salt or metallo-complex thereof for use according to claim 1, wherein the prion disease is selected from Creutzfeldt-Jakob diseases (CJD), Gerstmann-Sträussler-Scheinker syndrome (GSS), fatal familial insomnia (FFI), sporadic fatal insomnia (sFI), Variably Protease-Sensitive Prionopathy (VPSPr), kuru, scrapie, transmissible mink encephalopathy (TME), chronic wasting disease (CWD), bovine spongiform encephalopathy (BSE) and feline spongiform encephalopathy (FSE).

3. A pharmaceutical composition for use in the treatment and/or prevention of a prion disease, said composition comprising uroporphyrin I (UROI), or a pharmaceutically acceptable salt or metallo-complex thereof, and a pharmaceutically acceptable excipient.

4. The pharmaceutical composition for use according to claim 3, wherein the prion disease is selected from Creutzfeldt-Jakob diseases (CJD), Gerstmann-Sträussler-Scheinker syndrome (GSS), fatal familial insomnia (FFI), sporadic fatal insomnia (sFI), Variably Protease-Sensitive Prionopathy (VPSPr), kuru, scrapie, transmissible mink encephalopathy (TME), chronic wasting disease (CWD), bovine spongiform encephalopathy (BSE) and feline spongiform encephalopathy (FSE).

## Patentansprüche

1. Uroporphyrin I (UROI) oder ein pharmazeutisch akzeptables Salz oder ein Metallokomplex davon zur Verwendung bei der Behandlung und/oder Vorbeugung einer Prionenkrankheit.

2. Uroporphyrin I oder ein pharmazeutisch akzeptables Salz oder ein Metallokomplex davon zur Verwendung nach Anspruch 1, wobei die Prionenkrankheit ausgewählt ist aus den Creutzfeldt-Jakob-Krankheiten (CJD), dem Gerstmann-Sträussler-Scheinker-Syndrom (GSS), der tödlichen familiären Insomnie (FFI), der sporadischen tödlichen Insomnie (sFI), der variabel proteasesensitiven Prionopathie (VPSPr), Kuru, Scrapie, der übertragbaren Nerzenzephalopathie (TME), der chronischen Auszehrungskrankheit (CWD), der bovinen spongiformen Enzephalopathie (BSE) und der felinen spongiformen Enzephalopathie (FSE).

3. Eine pharmazeutische Zusammensetzung zur Verwendung bei der Behandlung und/oder Vorbeugung einer Prionenkrankheit, wobei die Zusammensetzung Uroporphyrin I (UROI) oder ein pharmazeutisch akzeptables Salz oder einen Metallokomplex davon sowie einen pharmazeutisch akzeptablen Hilfsstoff umfasst.

4. Die pharmazeutische Zusammensetzung zur Verwendung nach Anspruch 3, wobei die Prionenkrankheit ausgewählt ist aus den Creutzfeldt-Jakob-Krankheiten (CJD), dem Gerstmann-Sträussler-Scheinker-Syndrom (GSS), der tödlichen familiären Insomnie (FFI), der sporadischen tödlichen Insomnie (sFI), der variabel proteasesensitiven Prionopathie (VPSPr), Kuru, Scrapie, der übertragbaren Nerzenzephalopathie (TME), der chronischen Auszehrungskrankheit (CWD), der bovinen spongiformen Enzephalopathie (BSE) und der felinen spongiformen Enzephalopathie (FSE).

## Revendications

1. L'uroporphyrine I (UROI) ou un sel pharmaceutiquement acceptable ou un métallocomplexe de celle-ci pour une utilisation dans le traitement et/ou la prévention d'une maladie à prions.

2. L'uroporphyrine I ou un sel pharmaceutiquement acceptable ou un métallocomplexe de celle-ci pour une utilisation selon la revendication 1, dans laquelle la maladie à prions est choisie parmi les maladies de Creutzfeldt-Jakob (CJD), le syndrome de Gerstmann-Sträussler-Scheinker (GSS), l'insomnie fatale familiale (FFI), l'insomnie fatale sporadique (sFI), la prionopathie à sensibilité variable aux protéases (VPSPr), le kuru, la tremblante du mouton, l'encéphalopathie transmissible du vison (TME), la maladie du dépérissement chronique (CWD), l'encéphalopathie spongiforme bovine (BSE) et l'encéphalopathie spongiforme féline (FSE).

3. Une composition pharmaceutique pour une utilisation dans le traitement et/ou la prévention d'une maladie à prions, ladite composition comprenant l'uroporphyrine I (UROI), ou un sel pharmaceutiquement acceptable ou un métallocomplexe de celle-ci, et un excipient pharmaceutiquement acceptable.

4. La composition pharmaceutique pour une utilisation selon la revendication 3, dans laquelle la maladie à prions est choisie parmi les maladies de Creutzfeldt-Jakob (CJD), le syndrome de Gerstmann-Sträussler-Scheinker (GSS), l'insomnie fatale familiale (FFI), l'insomnie fatale sporadique (sFI), la prionopathie à sensibilité variable aux protéases (VPSPr), le kuru, la tremblante du mouton, l'encéphalopathie transmissible du vison (TME), la maladie du dépérissement chronique (CWD), l'encéphalopathie spongiforme bovine (BSE) et l'encéphalopathie spongiforme féline (FSE).
